**Europäisches Patentamt**

**European Patent Office**

**Office européen des brevets**

(19)

(11) Veröffentlichungsnummer: **0 135 058**
**B1**

(12) # EUROPÄISCHE PATENTSCHRIFT

(45) Veröffentlichungstag der Patentschrifft:
**17.01.90**

(21) Anmeldenummer: **84108705.9**

(22) Anmeldetag: **24.07.84**

(51) Int. Cl. ⁴: **A 61 K  7/00, A 61 K  47/00**

(54) **Verfahren zur Herstellung einer Trägermasse für kosmetische und/oder pharmazeutische Cremes.**

(30) Priorität: **29.07.83 DE 3327375**

(43) Veröffentlichungstag der Anmeldung:
**27.03.85 Patentblatt 85/13**

(45) Bekanntmachung des Hinweises auf die Patenterteilung:
**17.01.90 Patentblatt 90/03**

(84) Bennante Vertragsstaaten:
**AT BE CH FR GB IT LI LU NL SE**

(56) Entgegenhaltungen:
**AU-B-37 432**
**US-A-1 809 082**

**H.v. CZETSCH-LINDENWALD et al.: "Salben und Salbengrundlagen", 1939, Seite 203, Verlag von Julius Springer, Berlin, DE;**
**H.v. CZETSCH-LINDENWALD et al.: "Salben und Salbengrundlagen", 1939, Seiten 161-165, Verlag von Julius Springer, Berlin, DE;**
**"Hagers Handbuch der pharmazeutischen Praxis", 4. Ausgabe, Band VII, Teil B, 1977, Seiten 414-416, Springer-Verlag, Berlin, DE;**
**Christen "Grundlagen der organischen Chemie", 2. Auflage 1972, Seite 253**
**Hunting "Encyclopedia of Shampoo Ingredients" Seite 297**
**Römpps Chemie-Lexikon, Seite 3043 "Salben"**

(73) Patentinhaber: **Bartz, Peter**
**Von Bongardstrasse 13**
**D-5180 Eschweiler (DE)**

(72) Erfinder: **Bartz, Peter**
**Von Bongardstrasse 13**
**D-5180 Eschweiler (DE)**

(74) Vertreter: **Rauh, Wolfgang K., Dipl.-Ing. Patentanwalt**
**Mittelstrasse 55**
**D-5100 Aachen (DE)**

(56) Entgegenhaltungen: (fortsetzung)
**Janistyn "Handbuch der Kosmetika und Riechstoffe" I Band, Seite 656**
**Werr "Medizinische Klinik 1937, Seite 239**
**"Deutsches Lebensmittelbuch" 9. Lieferung, 9)1984 von Frank-Nüse, Abschn. C XI 1/9-1-14**
**Voigt/Bornschein "Lehrbuch der pharmazeutischen Technologie" S.267, Verlag Chemie**
**Herstellvorschrift für Catamin fettfrei**
**Schreiben der Fa. Riedel-de Haen bezüglich Catamin-Salbe vom 6.1.89**

**Die Akte enthält technische Angaben, die nach dem Eingang der Anmeldung eingereicht wurden und die nicht in dieser Patentschrift enthalten sind.**

EP 0 135 058 B1

## Beschreibung

Die Erfindung betrifft ein Verfahren zur Herstellung einer Trägermasse für kosmetische und/oder pharmazeutische Cremes mit Extrakten oder dgl. aus spurenelementenreichen Kräutern, insbesondere für die Regeneration der menschlichen Haut.

Für die Behandlung der Haut sind pharmazeutische Cremes, Salben oder Pasten bekannt, die aus einer Salbengrundlage oder Trägermasse mit Zinkoxid und Kolloidalem Schwefel und ggf. weiteren Wirkstoffen in unterschiedlichen Zusammensetzungen bestehen.

Aus Cetzsch-Lindenwald "Salben und Salbengrundlagen" S.163, Springer Verlag 1939 ist z. B. eine "Cathaminsalbe" bekannt, die 5 % kolloidalen Schwefel und 10 % Zinkoxid in neutraler Salbengrundlage enthält. Diese Salbengrundlage besteht laut Auskunft der Herstellerin im wesentlichen aus Wasser (72 % der Gesamtmenge) und Glyzerin (5 % der Gesamtmenge) und 3 - 3,5 % Sichozell. Diese Stoffe werden zunächst zu einer Paste verknetet und mit Zinkoxid gemischt. Wenn die Masse homogen geworden ist, gibt man Cosan 10 %, eine Lösung mit kolloidalem Schwefel zu und läßt die Masse weiterkneten und später über eine Dreiwalzenreibmaschine laufen.

Diese Salbe enthält somit nur einen sehr geringen Fettanteil (Glyzerin) und wird bei Raumtemperatur hergestellt. Sie wurde für Ekzeme eingesetzt und hat wegen der Verbindung kolloidalen Schwefels mit Zinkoxid auch therapeutische Erfolge bewirkt. Sie wird heute nicht mehr hergestellt.

Nach Auffassung der Dermatologen ist die Haut kein Verdauungsorgan, und gibt es folglich keine die Haut "ernährende" Cremes. Trotzdem ist es erforderlich, die Hautzellen mit den zu ihrer Erhaltung notwendigen Wirkstoffen zu versorgen, insbesondere dann, wenn durch Stoffwechselstörungen bereits Hautschädigungen oder -veränderungen eingetreten sind.

Um hier Abhilfe zu schaffen, ist es notwendig, eine Creme zu entwickeln, die nicht nur auf die Haut auftragbar ist und von dieser angenommen wird, sondern die auch in der Lage ist, die in ihr befindlichen, den Stoffwechsel der Haut ausgleichenden Spurenelemente in die Hautzellen, das Hautuntergewebe, die Blutbahn oder in das Muskelgewebe einzuführen und so aufzubrechen, daß diese Stoffe von der Haut angenommen, d.h. praktisch doch "verdaut" werden.

Eine wesentliche Voraussetzung hierzu ist die Herstellung einer geeigneten Trägermasse, die ein Oxidationsmittel für die zuzuführenden Spurenelemente bildet und diese in einen Zustand versetzt, in dem sie von der Haut voll aufgenommen werden.

Diese, der Erfindung zugrundeliegende Aufgabe wird dadurch gelöst, daß man eine Mischung aus

10 - 40 % aus pflanzlichen oder tierischen Fetten gewonnenem stearinarmem oder stearinfreiem Öl,

5 - 15 % kolloidalem Schwefel (in einer Konzentration bis 95 %),

10 - 40 % Zinkoxid und als

Rest Wasser von maximal 7° Härte bei einer Temperatur von 60 - 600°C unter Luftsauerstoffabschluß emulgiert und anschließend unter Zusatz eines bekannten Homogenisators homogenisiert und zu einer pastösen Masse abkühlt.

Der kolloidale Schwefel dient dazu, die der Haut zuzuführenden Stoffe ähnlich einem Verdauungsvorgang aufzubrechen und so aufzuarbeiten, daß sie von den Hautzellen aufgenommen werden. Um dies zu erreichen, wird der Schwefel mit Zinkoxid abgepuffert.

Ferner ist es wichtig, daß diese Stoffe möglichst kurzfristig und unter Luftsauerstoffabschluß, z. B. in einem Autoklaven, erhitzt, emulgiert und dann beim Abkühlen homogenisiert werden. Hierfür eignet sich Öl als besonders guter Wärmeträger, hinzu kommt, daß aus stearinfreiem bzw. -armem Fett hergestelltes Öl eine besonders hautfreundliche, und insbesondere die äußeren Hautschichten aufweichende Wirkung hat, die die Aufnahme der Wirkstoffe und deren Transport in die Hautzellen begünstigt. Diese Wirkung wird von stearinreichen Ölen nicht erreicht.

Je nach der Art der Stoffwechselstörung, z. B. Schuppenflechte, Schweißfuß, Akne, Porenverstopfung und dgl. kann der abkühlenden Trägermasse ein Sud oder Extrakt aus bestimmten spurenelementenreichen Kräutern zugemischt werden, um eine gezielt anwendbare Creme zu erhalten.

Diese Trägermasse bewirkt eine optimale, ballastfreie Aufnahme der Wirkstoffe aus den Kräuterextrakten und ermöglicht ferner eine hautfreundliche Abgabe der Wirkstoffe an die Haut und den Transport dieser Wirkstoffe in das Zellsystem der Haut in einer bereits aufgebrochenen Form.

Versuche haben gezeigt, daß aus dieser Trägermasse hergestellte Cremes außergewöhnlich gute Erfolge bei der Regeneration der menschlichen Haut und Beseitigung von stoffwechselbedingten Hautschäden erbracht haben.

## Patentanspruch

Verfahren zur Herstellung einer Trägermasse für kosmetische und/oder pharmazeutische Cremes, mit Extrakten aus spurenelementenreichen Kräutern, insbesondere für die Regeneration der menschlichen Haut, *dadurch gekennzeichnet*, daß man eine Mischung aus

10 - 40 % aus pflanzlichen oder tierischen Fetten gewonnenem stearinarmem oder stearinfreiem Öles,

5 - 15 % kolloidalem Schwefel (in einer Konzentration bis 95 %),

10 - 40 % Zinkoxid und als Rest Wasser von maximal 7° Härte bei einer Temperatur von 60 - 600°C unter Luftsauerstoffabschluß emulgiert und anschließend unter Zusatz eines bekannten Homogenisators homogenisiert und zu einer pastösen Masse abkühlt.

**Revendication**

Procédé de préparation d'un support pour des crèmes cosmétiques et/ou pharmaceutiques, avec des extraits d'herbes riches en oligo-éléments, en particulier pour la régénération de la peau humaine, caractérisé en ce qu'un mélange de:

10 - 40 % d'huile pauvre en stéarine ou sans stéarine, extraite de matières grasses animales ou végétales,
5 - 15 % de soufre colloïdal (en une concentration jusqu'à 95 %),
10 - 40 % d'oxyde de zinc, et,

comme restant, de l'eau d'une dureté maximale de 7°, est émulsifié à une température de 60 - 600°C en étant isolé de l'oxygène atmosphérique et ensuite homogénéisé en ajoutant un homogénéisateur connu, et refroidi en une masse pâteuse.

**Claim**

Process for preparing a carrier substance for cosmetic and/or pharmaceutical creams, utilising extracts from herbs which are rich in trace elements, more especially for the regeneration of human skin, characterised in that a mixture, comprising

10 - 40 % oil, which has been obtained from vegetable or animal fats and is low in stearin or free of stearin,
5 - 15 % colloidal sulphur (in a concentration up to 95 %),
10 - 40 % zinc oxide and, as the remainder, water with a maximum hardness of 7°, is emulsified at a temperature of 60 - 600°C with the exclusion of atmospheric oxygen, then it is homogenised by the addition of a known homogenising agent and cooled to form a pasty substance.